# EUROPEAN PATENT APPLICATION

(11) **EP 4 481 748 A1**
(43) Date of publication of application: **25.12.2024**
(21) Application number: 24180319.6
(22) Date of filing: 05.06.2024
(51) Int. Cl.: G16C 60/00, G16C 20/30, G16C 20/70

(54) **METHOD FOR PREDICTING PHYSICAL PROPERTIES OF POLYMERS AND POLYMER**

(30) Priority: 19.06.2023 JP 2023100052
(71) Applicant: Sumitomo Rubber Industries, Ltd., Kobe-shi, Hyogo 651-0072 (JP)
(72) Inventor: MINAGAWA, Yasuhisa, Kobe-shi, 651-0072 (JP)
(74) Representative: Manitz Finsterwald Patent- und Rechtsanwaltspartnerschaft mbB

(57) **Abstract**

Provided are a highly accurate method for predicting a physical property of a polymer and a polymer having excellent physical properties such as an ability to selectively capture specific cells and biocompatibility. The method for predicting a physical property of a polymer includes Step 1 of obtaining a structure of a monomer from a structure of a polymer, Step 2 of converting the obtained structure of the monomer or a converted structure of the monomer into a format recognizable by a computer, Step 3 of computing a descriptor from the format recognizable by a computer, Step 4 of obtaining a physical property of the polymer and performing a regression calculation using the physical property of the polymer as an objective variable and the descriptor as an explanatory variable to construct a regression model, Step 5 of computing a format of a new monomer from the format recognizable by a computer, Step 6 of computing a descriptor from the format of the new monomer or a converted format of the new monomer, and Step 7 of applying the descriptor of the new monomer to the regression model to predict a physical property of a polymer produced by polymerizing the new monomer.

## Description

### TECHNICAL FIELD

The present invention relates to a method for predicting a physical property of a polymer and a polymer.

### BACKGROUND ART

A technique to predict the physical properties of a new compound is proposed. The technique includes constructing a regression model for predicting the physical properties of a compound by machine learning, and predicting the physical properties of a new compound based on the regression model. The technique also includes repetition of a cycle of synthesizing a new compound, measuring the physical properties of the new compound to validate the predicted values, and adding the new compound and the actually measured physical properties to the data to improve the accuracy of the regression model. Further, the new compounds and their physical properties are utilized to discover useful compounds. Such a technique is popularly performed in the pharmaceutical field.

At present, however, the technique is not sufficiently used in the field of polymeric compounds (polymers), particularly in the prediction of specific physical properties (for example, moisture content, interstitial water content, unfreezable water content, adhesion to specific cells including cancer cells and stem cells, and biocompatibility) of polymers.

A substrate coated with a special polymer has been proposed as a device for capturing specific cells (e.g., blood cells, cancer cells present in blood or biological fluid) from blood or biological fluid (see, Patent Literature 1).

Unfortunately, the substrate may capture blood cells together with specific cells including cancer cells. There has been a demand for the development of polymers capable of selectively capturing specific cells including cancer cells while reducing the capture of normal cells such as blood cells.

Polymers with a better biocompatibility are also requested.

### CITATION LIST

### PATENT LITERATURE

Patent Literature 1: JP 2005-523981 T

### SUMMARY OF INVENTION

### TECHNICAL PROBLEM

The present invention aims to solve the above problem and provide a highly accurate method for predicting a physical property of a polymer and a polymer having excellent physical properties such as an ability to selectively capture specific cells and biocompatibility.

### SOLUTION TO PROBLEM

The present invention relates to a method for predicting a physical property of a polymer, the method including
Step 1 of obtaining a structure of a monomer from a structure of a polymer,
Step 2 of converting the obtained structure of the monomer or a converted structure of the monomer into a format recognizable by a computer,
Step 3 of computing a descriptor from the format recognizable by a computer,
Step 4 of obtaining a physical property of the polymer and performing a regression calculation using the physical property of the polymer as an objective variable and the descriptor as an explanatory variable to construct a regression model,
Step 5 of computing a format of a new monomer from the format recognizable by a computer,
Step 6 of computing a descriptor from the format of the new monomer or a converted format of the new monomer, and
Step 7 of applying the descriptor of the new monomer to the regression model to predict a physical property of a polymer produced by polymerizing the new monomer.

### ADVANTAGEOUS EFFECTS OF INVENTION

The method for predicting a physical property of a polymer of the present invention includes the Steps 1 to 7, and thus the present invention can provide a highly accurate method for predicting a physical property of a polymer. The method is expected to enable rapid discovery of polymers (candidate polymers) with many excellent physical properties.

Moreover, the present invention is expected to provide a polymer having excellent physical properties such as an ability to selectively capture specific cells including cancer cells and biocompatibility.

### BRIEF DESCRIPTION OF DRAWINGS

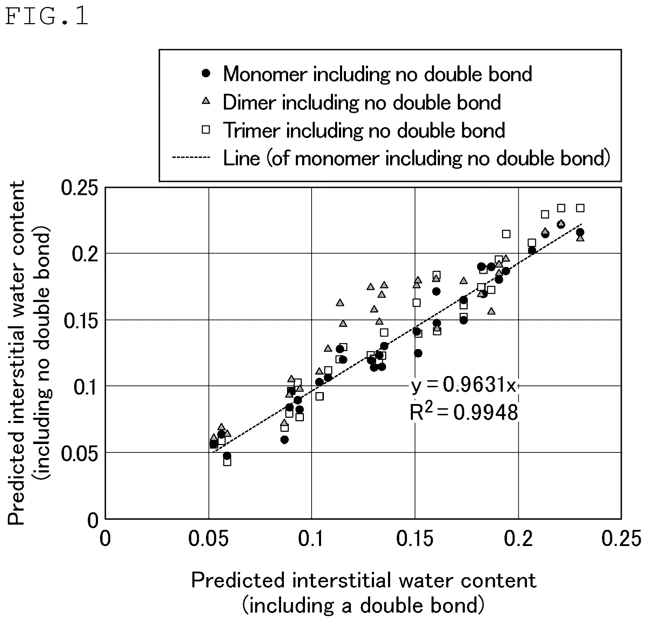

FIG. 1 is a diagram showing the relationships between predicted interstitial water contents of the structures of 33 new monomers and predicted interstitial water contents of compounds converted from the structures of the monomers based on a regression model constructed in Step 4.

### DESCRIPTION OF EMBODIMENTS

### <Method for predicting physical property of polymer>

The method for predicting a physical property of a polymer of the present invention includes Step 1 of obtaining a structure of a monomer from a structure of a polymer, Step 2 of converting the obtained structure of the monomer or a converted structure of the monomer into a format recognizable by a computer, Step 3 of computing a descriptor from the format recognizable by a computer, Step 4 of obtaining a physical property of the polymer and performing a regression calculation using the physical property of the polymer as an objective variable and the descriptor as an explanatory variable to construct a regression model, Step 5 of computing a format of a new monomer from the format recognizable by a computer, Step 6 of computing a descriptor from the format of the new monomer or a converted format of the new monomer, and Step 7 of applying the descriptor of the new monomer to the regression model to predict a physical property of a polymer produced by polymerizing the new monomer. In each of steps 3, 5 and 6, the computing is preferably carried out by a computer.

The method for predicting a physical property of a polymer includes Step 1 of obtaining a structure of a monomer from a structure of a polymer. For example, in the Step 1, the structure of a monomer having a carbon-carbon double bond which constitutes a polymer is obtained from the structure of the polymer. Specifically, when the structure of the polymer is polyacrylic acid, acrylic acid, which is a structure of a monomer constituting the polyacrylic acid, is obtained.

The polymer in the Step 1 is not limited and may be an appropriate known polymer.

Examples of the polymer include homopolymers of a single monomer and copolymers of two or more monomers. The polymer may be used alone or in combinations of two or more.

The polymer can be produced by known methods. For example, it may be synthesized by polymerizing a monomer constituting the polymer using a solution of the monomer by a known method. Non-limiting examples of the solvent of the monomer solution include those described later. Toluene or methanol is preferred among those.

From the standpoint of an ability to selectively capture specific cells including cancer cells and biocompatibility, the polymer is desirably a polymer with hydrophilicity (hydrophilic polymer).

Examples of the hydrophilic polymer include homopolymers or copolymers of one or two or more hydrophilic monomers, and copolymers of one or two or more hydrophilic monomers and one or two or more different monomers. Each of these may be used alone or in combinations of two or more.

Non-limiting examples of the hydrophilic monomer include various monomers containing hydrophilic groups. Examples of the hydrophilic groups include known hydrophilic groups such as an amide group, a sulfuric acid group, a sulfonic acid group, a carboxylic acid group, a hydroxy group, an amino group, and an oxyethylene group.

Specific examples of the hydrophilic monomer include (meth)acrylic acids, (meth)acrylic acid esters, alkoxyalkyl (meth)acrylates such as methoxyethyl (meth)acrylates, hydroxyalkyl (meth)acrylates such as hydroxyethyl (meth)acrylates, (meth)acrylamides, and (meth)acrylamide derivatives containing cyclic groups such as (meth)acryloylmorpholines. From the standpoint of an ability to selectively capture specific cells including cancer cells and biocompatibility, (meth)acrylic acids, (meth)acrylic acid esters, and alkoxyalkyl (meth)acrylates are preferred, alkoxyalkyl (meth)acrylates are more preferred, and 2-methoxyethylacrylate is particularly preferred among these. Each of these may be used alone or in combinations of two or more.

The different monomer may be appropriately selected from those which do not inhibit the effect of the hydrophilic polymer. Specific examples of the different monomer include aromatic monomers such as styrene, vinyl acetate, and N-isopropylacrylamide which can impart temperature responsiveness. Each of these may be used alone or in combinations of two or more.

Specific examples of the homopolymers and copolymers include: homopolymers formed from a single hydrophilic monomer, such as polyacrylic acids, polyacrylic acid esters, polymethacrylic acids, polymethacrylic acid esters, polyacryloylmorpholine, polymethacryloylmorpholine, polyacrylamide, polymethacrylamide, polyalkoxyalkyl acrylate, and polyalkoxyalkyl methacrylate; copolymers formed from two or more hydrophilic monomers listed above; and copolymers formed from one or more hydrophilic monomers listed above and one or more different monomers listed above. Each of these hydrophilic polymers may be used alone or in combinations of two or more.

Specific examples of homopolymers and copolymers of the hydrophilic monomer include polyacrylic acids, polyacrylic acid esters, polymethacrylic acid, polymethacrylic acid esters, polyacryloyl morpholine, polymethacryloyl morpholine, polyacrylamide, polymethacrylamide, polyalkoxyalkyl acrylate, and polyalkoxyalkyl methacrylate.

From the standpoint of an ability to selectively capture specific cells including cancer cells and biocompatibility, the hydrophilic polymer is preferably a polymer represented by the following formula (I): 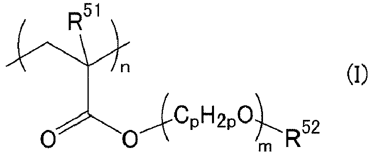 wherein R⁵¹ represents a hydrogen atom or a methyl group; R⁵² represents an alkyl group; p represents 1 to 8; m represents 1 to 5; and n represents the number of repetitions. The polymer may be used alone or in combinations of two or more.

From the standpoint of an ability to selectively capture specific cells including cancer cells and biocompatibility, the polymer represented by the formula (I) is preferably a polymer represented by the following formula (I-1): 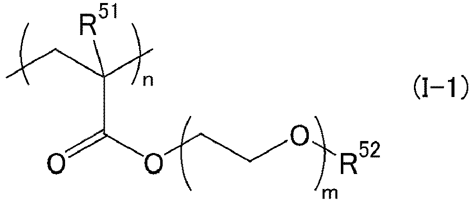 wherein R⁵¹ represents a hydrogen atom or a methyl group; R⁵² represents an alkyl group; m represents 1 to 5; and n represents the number of repetitions. The polymer may be used alone or in combinations of two or more.

The number of carbon atoms of the alkyl group for R⁵² is preferably 1 to 10, more preferably 1 to 5. R⁵² is particularly preferably a methyl group or an ethyl group. The symbol p is preferably 1 to 5, more preferably 1 to 3. The symbol m is preferably 1 to 3. The symbol n (the number of repeating units) is preferably 15 to 1500, more preferably 40 to 1200.

From the standpoint of an ability to selectively capture specific cells including cancer cells and biocompatibility, the hydrophilic polymer is preferably a copolymer of a compound represented by the following formula (II) with a different monomer. The copolymer may be used alone or in combinations of two or more.

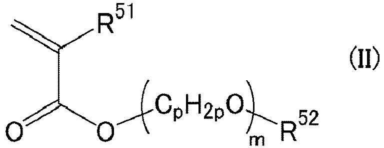

In the formula, R⁵¹, R⁵², p, and m are as defined above.

From the standpoint of an ability to selectively capture specific cells including cancer cells and biocompatibility, the polymer represented by the formula (II) is preferably a compound represented by the following formula (II-1): 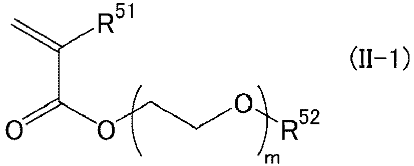 wherein R⁵¹, R⁵², and m are as defined above. The compound may be used alone or in combinations of two or more.

From the standpoint of an ability to selectively capture specific cells including cancer cells and biocompatibility, a hydrophilic polymer represented by the formula (I) is preferred, a hydrophilic polymer represented by the formula (I-1) is more preferred, and polyalkoxyalkyl acrylate or polyalkoxyalkyl methacrylate is still more preferred among the above-described hydrophilic polymers.

To better achieve the advantageous effect, the number average molecular weight (Mn) of the polymer is preferably 8000 to 150000, more preferably 10000 to 60000, still more preferably 10000 to 39000.

Herein, the number average molecular weight (Mn) can be determined with a gel permeation chromatograph (GPC) (GPC-8000 series available from Tosoh Corporation, detector: differential refractometer, column: TSKgel SuperMultipore HZ-M available from Tosoh Corporation). The resulting value is calibrated with polystyrene standards.

In the Step 1 in the method for predicting a physical property of a polymer, when the structure of the polymer is poly(2-methoxyethyl acrylate), for example, 2-methoxyethyl acrylate (a structure of a monomer) is obtained.

After the Step 1 in the method for predicting a physical property of a polymer, Step 2 is performed in which the obtained structure of the monomer or a converted structure of the monomer is converted into a format recognizable by a computer. Specifically, in the Step 2, a compound with the structure of the monomer obtained in the Step 1 (the original structure of the monomer obtained) or a compound converted from the structure of the monomer is converted into a format recognizable by a computer.

The structure of the monomer obtained in the Step 1 may be converted by any technique that can convert the structures of monomers, such as the following techniques (1) and (2): a technique (1) of cleaving a carbon-carbon double bond in the structure of the monomer obtained in the Step 1 (the original structure of the monomer including a carbon-carbon double bond) and
introducing a hydrogen atom or a saturated hydrocarbon group such as a methyl group to each terminal of the cleaved monomer to form a compound (converted into a monomer including no carbon-carbon double bond); and a technique (2) of cleaving a carbon-carbon double bond in the structure of the monomer obtained in the Step 1 (the original structure of the monomer) to form a multimer containing only carbon-carbon single bonds and introducing a hydrogen atom or a saturated hydrocarbon group such as a methyl group to each terminal of the multimer to form a compound (converted into a multimer including no carbon-carbon double bond, such as a dimer including no carbon-carbon double bond or a trimer including no carbon-carbon double bond). A desirable technique in the case of conversion to the multimer includes cleaving a carbon-carbon double bond in the structure of the monomer obtained in the Step 1 (the original structure of the monomer) to form a multimer containing only carbon-carbon single bonds and introducing a methyl group to each terminal of the multimer to form a compound.

In the step 2, a compound with the structure of the monomer (the original structure of the monomer) obtained in the Step 1 or a multimer including no carbon-carbon double bond converted from the structure of the monomer is converted into a format recognizable by a computer. Examples of the computer include usual personal computers, servers, workstations, compute nodes, and microcomputers. The hardware of the computer may be a known one.

Examples of the format recognizable by a computer include known formats such as a simplified molecular input line entry system (SMILES) format and a MOL file format.

In the Step 2 in the method for predicting a physical property of a polymer, when the structure of the polymer is, for example, poly(2-methoxyethyl acrylate), the 2-methoxyethyl acrylate (the original structure of the monomer including a carbon-carbon double bond) obtained in the Step 1 is used as it is or one of the following compounds (A) to (C) converted from the 2-methoxyethyl acrylate is used.
(A) A monomer including no carbon-carbon double bond: a compound produced by cleaving the carbon-carbon double bond of the 2-methoxyethyl acrylate and introducing a methyl group to each terminal of the monomer
(B) A dimer including no carbon-carbon double bond: a compound produced by cleaving the carbon-carbon double bond of the 2-methoxyethyl acrylate to form a dimer containing only carbon-carbon single bonds and introducing a methyl group to each terminal of the dimer
(C) A trimer including no carbon-carbon double bond: a compound produced by cleaving the carbon-carbon double bond of the 2-methoxyethyl acrylate to form a trimer containing only carbon-carbon single bonds and introducing a methyl group to each terminal of the trimer

In the Step 2, the 2-methoxyethyl acrylate or one of the compounds (A) to (C) is converted into one of the following SMILES formats (SMILES notations) which are formats recognizable by a computer.

2-methoxyethyl acrylate: C=C(C(=O)OCCOC) (hydrogen atoms are not shown, parentheses indicate branches)
(A) a monomer including no carbon-carbon double bond: CCC(C(=O)OCCOC)C
(B) a dimer including no carbon-carbon double bond: CCC(C(=O)OCCOC)CC(C(=O)OCCOC)C
(C) a trimer including no carbon-carbon double bond: CCC(C(=O)OCCOC)CC(C(=O)OCCOC)CC(C(=O)OCCOC)C

The method for predicting a physical property of a polymer includes the Step 2 and separately Step 3 of computing a descriptor from the format recognizable by a computer. Specifically, the structure of the monomer including a carbon-carbon double bond (the original structure of the monomer including a carbon-carbon double bond) constituting a polymer with known physical properties, structure, etc. is converted into a format recognizable by a computer. Alternatively, a compound converted from the structure of the monomer (a multimer including no carbon-carbon double bond, such as the monomer including no carbon-carbon double bond, the dimer including no carbon-carbon double bond, or the trimer including no carbon-carbon double bond) is converted into a format recognizable by a computer. Then, a descriptor is computed from the format.

Non-limiting examples of the physical property of the polymer include glass transition temperature (Tg), melting point, refractive index, contact angle, surface free energy, density, water absorption, interstitial water content, unfreezable water content, selective adhesion to specific cells including cancer cells, and biocompatibility. Of these, the method can suitably predict interstitial water content, selective adhesion to specific cells, and biocompatibility.

In the Step 3, the original structure of the monomer constituting the polymer or the compound converted from the structure of the monomer is converted into a format recognizable by a computer. Examples of the format include the SMILES format and the MOL file format described above. A descriptor (numerical data) to numerically represent the chemical structure is computed from the format. For example, in Python, 208 descriptors or 400 descriptors are computed by RDkit from SMILES. The descriptors can also be computed using mordred, PaDEL, alvaDesc, etc. Fingerprints may be another method to numerically represent the chemical structure.

The step 3 in the method for predicting a physical property of a polymer includes preparing a SMILE format of the structure of the monomer (the original structure of the monomer including a carbon-carbon double bond) constituting one of 11 polymers with known interstitial water contents or a SMILE format of a compound converted from the structure of the monomer (the monomer including no carbon-carbon double bond, the dimer including no carbon-carbon double bond, or the trimer including no carbon-carbon double bond). From each SMILE format, it is possible to compute 208 descriptors of the numerical data reflecting the chemical structure.

The Step 3 may be performed separately from the Steps 1 and 2. The Steps 1 and 2 and the Step 3 may be performed in any order. For example, the Step 3 may be performed after the Steps 1 and 2, simultaneously with the Steps 1 and 2, or before the Steps 1 and 2.

In the method for predicting a physical property of a polymer, Step 4 of obtaining a physical property of the polymer and performing a regression calculation using the physical property of the polymer as an objective variable and the descriptor as an explanatory variable to construct a regression model is performed after the Steps 1, 2, and 3. Specifically, in the Step 4, a regression formula (regression model) is constructed by regression calculation using the physical property of the polymer as an objective variable and the descriptor (for example, 208 descriptors described above) as an explanatory variable.

Known algorithms used in machine learning and the like may be used for the model construction. The regression calculation to construct the regression formula (regression model) may be a known method such as multiple regression calculation, support vector regression calculation, decision tree calculation, random forest regression calculation, XGBoost regression calculation, LightGBM regression calculation, CatBoost regression calculation, Gaussian process regression calculation, neural networks, or deep learning. One regression calculation may be performed alone or a combination of two or more regression calculations may be performed.

In the Step 4 in the method for predicting a physical property of a polymer, four models to predict the interstitial water content can be constructed from four structures including the structure of the monomer (the original structure of the monomer including a carbon-carbon double bond) constituting the polymer and the structures of compounds converted from the structure of the monomer (the monomer including no carbon-carbon double bond, the dimer including no carbon-carbon double bond, and the trimer including no carbon-carbon double bond) using their interstitial water contents as objective variables and 208 descriptors as explanatory variables by ensemble learning including random forest, XGBoost regression calculation, LightGBM regression calculation, and CatBoost regression calculation.

In the case of Boost regression calculation, neural networks, or deep learning, adjustment of hyperparameters is performed to reduce overfitting and to allow the entire dataset usable for training. Examples of the technique for the adjustment of hyperparameters include grid research, random research, and Bayesian optimization. The term "hyperparameter" refers to a parameter that is determined by humans before training. Hyperparameters are necessary to control the behaviors of algorithms. Typical examples include a maximum depth of decision trees, a maximum number of leaves in decision trees, a minimum number of samples per leaf, a proportion of samples randomly extracted from each decision tree, a proportion of columns randomly extracted from each decision tree, a L1 regularization term for leaf weights in decision trees, a L2 regularization term for leaf weights in decision trees, a minimum loss reduction by adding leaves to decision trees, a method to optimize a loss function, the number of boosting rounds, the number of epochs, a learning rate, a threshold, a minibatch size, the number of layers, and the number of neurons per layer.

In the method for predicting a physical property of a polymer, Step 5 of computing the format of a new monomer from the format recognizable by a computer is performed after the Steps 1, 2, 3, and 4. Specifically, in the Step 5, the format of a new monomer is computed (generated) from the format, such as a SMILES format or a MOL file format, of the structure of the monomer (the monomer including a carbon-carbon double bond) constituting the polymer and the like.

The format recognizable by a computer of a new monomer can be computed (generated) by, for example, fragmenting the chemical structure of the compound in the format using the breaking of retrosynthetically interesting chemical substructures (BRICS) algorithm, the retrosynthetic combinatorial analysis procedure (RECAP) algorithm, the design of genuine structures (DOGS) algorithm, or the like and then mechanically combining the fragments. Examples of the format recognizable by a computer include the SMILES format and the MOL file format described above.

When using RECAP, for example, an organic molecule is broken into fragments at sites where bonds therein are easily cleaved. Examples of the bonds to consider include amide bonds, ester bonds, amine bonds, and N-C bonds in urea, ether bonds, C=C bonds, ammonia, N-S bonds in sulfenamide, aromatic ring-aromatic ring bonds, N (in an aromatic ring)-C (sp3) bonds, and N (in a lactam ring)-C (sp3) bonds. When using BRICS, an organic molecule is broken into fragments while considering 16 types of bonds by the same method as described for RECAP.

In the computation (generation) of the format recognizable by a computer of a new monomer, compounds including no carbon-carbon double bond may be generated. In order to predict the physical property of the polymer, compounds including no carbon-carbon double bond are excluded from the generated monomers.

In the Step 5 in the method for predicting a physical property of a polymer, for example, 74 compounds are generated using BRICS or the like from the SMILES formats of the structures of 11 monomers (the original structures of the monomers including a carbon-carbon double bond) respectively constituting 11 types of polymers each with a known interstitial water content. From the compounds, 33 compounds that can be monomers are selected (41 others are excluded). Thus, the SMILES formats of the 33 new monomers are computed (generated).

The method for predicting a physical property of a polymer includes Step 6 of computing a descriptor from the format (format recognizable by a computer) of the new monomer computed (generated) in the Step 5 or a converted format of the new monomer. Specifically, in the Step 6, first, the format (format recognizable by a computer) of the new monomer computed (generated) in the Step 5 is converted into a format of a compound with the structure of the monomer (the original structure of the monomer) or a format of a compound converted from the structure of the monomer by the same method as in the Step 2, and then a descriptor (numerical data) is computed from the format by the same method as in the Step 3.

In the Step 6 in the method for predicting a physical property of a polymer, for example, SMILES formats of the 33 new monomers computed (generated) as above are generated from the respective structures of the 33 new monomers (the original structures of the monomers including a carbon-carbon double bond) or the structures of compounds converted from the structures of the monomers (the monomer including no carbon-carbon double bond, the dimer including no carbon-carbon double bond, the trimer including no carbon-carbon double bond), and then descriptors (numerical data) are computed from the SMILES formats.

The method for predicting a physical property of a polymer includes Step 7 of applying the descriptor of the new monomer to the regression model to predict a physical property of a polymer produced by polymerizing the new monomer. Specifically, in the Step 7, the descriptor (numerical data) of the new monomer computed in the Step 6 is applied to the regression model constructed in the Step 4 to predict the physical property of a polymer produced by polymerizing the new monomer.

In the Step 7, for example, the descriptors (numerical data) of the structures of the 33 new monomers (the original structures of the monomers including a carbon-carbon double bond) or the compounds converted from the monomers (the monomers including no carbon-carbon double bond, the dimers including no carbon-carbon double bond, the trimers including no carbon-carbon double bond) computed (generated) in the Step 6 are applied to the regression model constructed in the Step 4 to predict the interstitial water contents of polymers respectively produced by polymerizing the new monomers.

Specifically, FIG. 1 shows the relationships between predicted interstitial water contents of the structures of polymers produced by polymerizing the structures of the 33 new monomers (the original structures of the monomers including a carbon-carbon double bond) and predicted interstitial water contents of polymers produced by polymerizing compounds converted from the structures of the monomers (the monomers including no carbon-carbon double bond, the dimers including no carbon-carbon double bond, the trimers including no carbon-carbon double bond) based on the regression model constructed in the Step 4. FIG. 1 shows that there is no difference in the interstitial water content between the case with a carbon-carbon double bond and the case with no carbon-carbon double bond and that an increase in the number of the monomers or multimers cause no difference.

Further, when compounds are generated while setting the maximum number of compounds to be generated to 2000 and also the compounds are generated from the structures of the monomers (the original structures of the monomers including a carbon-carbon double bond) derived from the 11 types of polymers using the BRIXCS algorithm, more than 1600 compounds (SMILES notations) are generated. From the generated compounds, compounds including no carbon-carbon double bond and compounds including two or more carbon-carbon double bonds are excluded to select 625 compounds including only one carbon-carbon double bond. The descriptors of the 625 compounds are computed. Then, the interstitial water contents of the 625 compounds are predicted from the prediction models of the compounds including only one carbon-carbon double bond.

The interstitial water contents from 0.01 to 0.15 among the predicted interstitial water contents are shown in Table 1-1, Table 1-2 (continued from Table 1-1), and Table 1-3 (continued from Table 1-1).

The interstitial water contents from 0.22 to 0.5 are shown in Table 2-1, Table 2-2 (continued from Table 2-1), Table 2-2 (continued from Table 2-1), Table 2-3 (continued from Table 2-1), Table 2-4 (continued from Table 2-1), Table 2-5 (continued from Table 2-1), Table 2-6 (continued from Table 2-1), and Table 2-7 (continued from Table 2-1).

**[Table 1-1]**

| Random forest | |
|---|---|
| Predicted interstitial water content | SMILES notation |
| 0.07128 | C=CC(=O)OCC1(C)COCOC1 |
| 0.0712 | C=CC(=O)OCC1(CC)COCOC1 |
| 0.06238 | C=CC(=O)OCCCOC |
| 0.05981 | C=CC(=O)OCC1CCCCO1 |
| 0.07843 | C=CC1COC(=O)O1 |
| 0.04277 | C=COC1CCCCO1 |

**[Table 1-2]**

| Predicted interstitial water content | SMILES notation |
|---|---|
| 0.05764 | C=CC(=O)OCCOC |
| 0.06074 | C=CC(=O)OCCOCC |
| 0.0645 | C=CC(=O)OCCCOC |
| 0.08501 | C=CC(=O)OCC |
| 0.08508 | C=COCC |
| 0.08885 | C=COCCOC |
| 0.09007 | C=CC(=O)OC |
| 0.09229 | C=CC(=O)OCCCOCC |
| 0.09312 | C=COCCOCC |
| 0.09454 | C=CC(=O)OCCOCCO |
| 0.09721 | C=COCCCOCC |
| 0.09967 | C=COCCO |
| 0.1038 | C=CC(=O)OCCCOCCO |
| 0.10466 | C=COCCCOC |
| 0.1071 | C=CC(=O)OCCO |
| 0.10898 | C=C(C)C(=O)OCCOC |
| 0.11156 | C=C(C)C(=O)OCC |
| 0.11367 | C=C(C)C(=O)OC |
| 0.11511 | C=COCCOCCOC |
| 0.11758 | C=COCCOCCO |
| 0.12047 | C=COCCCOCCO |
| 0.12118 | C=COCC1OCC(CC)O1 |
| 0.12379 | C=COCC1CCCO1 |
| 0.12577 | C=C(C)C(=O)OCCO |
| 0.12658 | C=COCC1CCCCO1 |
| 0.12665 | C=COC1COC(OC)O1 |
| 0.12753 | C=C(C)C(=O)OCCCOC |
| 0.12795 | C=COC(OCC)OCC |
| 0.12889 | C=COC1CCCO1 |
| 0.13072 | C=COC1COC(OCC)O1 |
| 0.13127 | C=COCCOCCCOC |
| 0.1326 | C=COC(OCC)OCCO |
| 0.13548 | C=COC1OCC(OC)O1 |
| 0.13605 | C=COCCCOCCOC |
| 0.13619 | C=COC1CC(OCC)01 |
| 0.13656 | C=COCCOC1CCCO1 |
| 0.13864 | C=COC1COC(CC)O1 |
| 0.13903 | C=COC1CCCCO1 |
| 0.13972 | C=COCC1OCC(CC)O1 |
| 0.13985 | C=COCC1(CC)COCOC1 |
| 0.14115 | C=COCCOCC1CCCO1 |
| 0.14262 | C=CC(=O)OC1CCCO1 |
| 0.14284 | C=COCC1COC(CC)O1 |
| 0.14287 | C=COC1(CC)COCOC1 |
| 0.14313 | C=C(C)C(=O)OCCCOCC |
| 0.14401 | C=COCCCOC1CCCO1 |
| 0.14447 | C=COC1COC(COC)O1 |
| 0.14576 | C=COC1OCC(CC)O1 |
| 0.14591 | C=COCC1COC(OC)O1 |
| 0.14654 | C=COC1(OC)COCOC1 |
| 0.14779 | C=COCCOC1CCCCO1 |
| 0.14811 | C=COC(OCC)OCCOC |

**[Table 1-3]**

| Predicted interstitial water content | SMILES notation |
|---|---|
| 0.1377 | C=C(C(=O)OCC(OC)OC) |
| 0.0618 | C=C(C(=O)OCCOC) |
| 0.1213 | C=C(C(=O)OCCOCCOCC) |
| 0.1091 | C=C(C(=O)OCCOCCOC) |
| 0.095 | C=C(C(=O)CCCC) |

**[Table 2-1]**

| Predicted interstitial water content | SMILES notation |
|---|---|
| 0.22003 | C=COC(OC1COC(OC)O1)OC1(C2CCCCO2)COCOC1 |
| 0.22003 | C=COC(OC1OCC(OC)O1)OC1(C2CCCCO2)COCOC1 |
| 0.22005 | C=COC(OC1CCCCO1)OC1(OC2CCCO2)COCOC1 |
| 0.22005 | C=COC(OC1CCCO1)OC1(OC2CCCCO2)COCOC1 |
| 0.22018 | C=COC(OCCO)OCC1(C2CCCCO2)COCOC1 |
| 0.22018 | C=COC(OCCO)OC1(C2CCCCO2)COCOC1 |
| 0.22018 | C=COC(OCCO)OC1COC(C2(C3CCCO3)COCOC2)O1 |
| 0.22018 | C=COC(OCCO)OCC1(C2CCCO2)COCOC1 |
| 0.22031 | C=COC1OCC(C2(OC3CCCCO3)COCOC2)O1 |
| 0.22031 | C=COC(OC1CCCO1)OC1(C2COC(CC)O2)COCOC1 |
| 0.22035 | C=COC(OC1CCCO1)OC1(OC2CCCO2)COCOC1 |
| 0.22037 | C=COC(OCC)OC1(C2CCCCO2)COCOC1 |
| 0.2206 | C=COC(OCCO)OC1OCC(C2(C3CCCO3)COCOC2)O1 |
| 0.22064 | C=COCCCOC1(C2CCCCO2)COCOC1 |
| 0.22064 | C=C(C)C(=O)OC1COC(OCC)O1 |
| 0.22067 | C=COC(OCC1CCCO1)OC1(CC)COCOC1 |
| 0.22071 | C=CC(=O)OC1COC(OC2CCCCO2)O1 |
| 0.22071 | C=COCCOC1(C2CCCCO2)COCOC1 |
| 0.22074 | C=COC(OC1OCC(CC)O1)OC1(C2CCCO2)COCOC1 |
| 0.22077 | C=COC(OCC)OCC1(C2CCCO2)COCOC1 |
| 0.2208 | C=COC(OC1CCCCO1)OC1(C2COC(CC)O2)COCOC1 |
| 0.22081 | C=COC(OC1CCCCO1)OC1(C2CCCO2)COCOC1 |
| 0.22081 | C=COC(OC1CCCO1)OC1(C2CCCCO2)COCOC1 |
| 0.22081 | C=COC(OC1CCCO1)OC1(C2CCCO2)COCOC1 |
| 0.22095 | C=COC(OC1COC(CC)O1)OC1(C2CCCO2)COCOC1 |
| 0.2211 | C=COC(OCCO)OC1COC(C2(C3CCCCO3)COCOC2)O1 |
| 0.22115 | C=COC(OC1CCCO1)OC1(C2OCC(CC)O2)COCOC1 |

**[Table 2-2]**

| | |
|---|---|
| 0.22116 | C=COC(OC1OCC(CC)O1)OC1(C2CCCCO2)COCOC1 |
| 0.22119 | C=COC1(OC2(OC)COCOC2)COCOC1 |
| 0.22137 | C=COC(OC1COC(CC)O1)OC1(C2CCCCO2)COCOC1 |
| 0.22142 | C=COC(OCC)OC1(C2OCC(CC)O2)COCOC1 |
| 0.22146 | C=COC(OCCOCCO)OC1CCCO1 |
| 0.22164 | C=COC(OC1CCCCO1)OC1(C2OCC(CC)O2)COCOC1 |
| 0.22178 | C=COC(OCCCOC)OC1COC(OC)O1 |
| 0.22214 | C=COC1(C2OCC(OCCO)O2)COCOC1 |
| 0.22214 | C=COC1COC(C2(OCCO)COCOC2)O1 |
| 0.22221 | C=COC(OC1COC(CC)O1)OC1COC(OC)O1 |
| 0.22228 | C=COC(OCCOC)OCC1CCCO1 |
| 0.22233 | C=COC(OCC)OCCCOC1CCCCO1 |
| 0.22239 | C=COCC1COC(C2(C3CCCCO3)COCOC2)O1 |
| 0.22239 | C=COCC1OCC(C2(C3CCCCO3)COCOC2)O1 |
| 0.22239 | C=COCC1OCC(C2(C3CCCO3)COCOC2)O1 |
| 0.22257 | C=COC(OC1CCCCO1)OC1OCC(COC)O1 |
| 0.22261 | C=CC(=O)OC1(OC2CCCCO2)COCOC1 |
| 0.22265 | C=COC(OCC)OCC1COC(OC)O1 |
| 0.22269 | C=COC(OC1CCCO1)OC1COC(OC2CCCO2)O1 |
| 0.22274 | C=COC(OCCCOCCO)OC1CCCO1 |
| 0.22278 | C=COC(OC1CCCO1)OC1OCC(C2(C3CCCO3)COCOC2)O |
| 0.22278 | C=COC(OC1CCCO1)OC1COC(C2(C3CCCO3)COCOC2)O |
| 0.22285 | C=COC(OCC)OC1(C2COC(CC)O2)COCOC1 |
| 0.22295 | C=COC1OCC(C2(C3CCCO3)COCOC2)O1 |
| 0.22302 | C=C(C)C(=O)OC1(OC)COCOC1 |
| 0.22308 | C=COC(OCC1CCCCO1)OC1(C2CCCO2)COCOC1 |
| 0.22308 | C=COC(OCC1CCCO1)OC1(C2CCCCO2)COCOC1 |
| 0.22308 | C=COC(OCC1CCCO1)OC1(C2CCCO2)COCOC1 |
| 0.22312 | C=COC(OC1CCCCO1)OC1OCC(OC2CCCCO2)O1 |
| 0.22312 | C=COC(OC1CCCCO1)OC1COC(OC2CCCCO2)O1 |
| 0.22313 | C=COC(OCCO)OCC1COC(OC)O1 |
| 0.22314 | C=COC(OCCO)OC1OCC(OCCO)O1 |
| 0.22314 | C=COC(OCCO)OC1COC(OCCO)O1 |
| 0.22315 | C=COC1OCC(C2(C3CCCCO3)COCOC2)O1 |
| 0.22321 | C=COC(OC1OCC(CC)O1)OC1(CC)COCOC1 |
| 0.22322 | C=COC(OCC)OC1COC(C2(C3CCCO3)COCOC2)O1 |
| 0.22327 | C=COC1(C2COC(OCCO)O2)COCOC1 |
| 0.22328 | C=COC(OCC1(C2CCCO2)COCOC1)OC1CCCCO1 |
| 0.22328 | C=COC(OC1CCCCO1)OC1OCC(C2(C3CCCO3)COCOC2)( |

**[Table 2-3]**

| | |
|---|---|
| 0.22328 | C=COC(OC1CCCCO1)OC1COC(C2(C3CCCO3)COCOC2)C |
| 0.22328 | C=COC(OC1CCCO1)OC1OCC(C2(C3CCCCO3)COCOC2)C |
| 0.22328 | C=COC(OCC1(C2CCCCO2)COCOC1)OC1CCCO1 |
| 0.22328 | C=COC(OC1CCCO1)OC1COC(C2(C3CCCCO3)COCOC2)C |
| 0.22328 | C=COC(OCC1(C2CCCO2)COCOC1)OC1CCCO1 |
| 0.22333 | C=COC(OC1CCCCO1)OC1(OC2CCCCO2)COCOC1 |
| 0.2234 | C=COC(OCCOC1CCCCO1)OC1CCCCO1 |
| 0.22345 | C=COC(OC1COC(CC)O1)OC1COC(CC)O1 |
| 0.22345 | C=COC(OCC1CCCCO1)OC1(CC)COCOC1 |
| 0.22351 | C=COC(OCC1CCCCO1)OC1(C2CCCCO2)COCOC1 |
| 0.22353 | C=COC(OCC1(OC)COCOC1)OC1CCCO1 |
| 0.22353 | C=COC(OC1CCCO1)OC1(COC)COCOC1 |
| 0.22362 | C=COC(OCC)OCC1(C2CCCCO2)COCOC1 |
| 0.22364 | C=COC(OCC)OC1COC(C2(C3CCCCO3)COCOC2)O1 |
| 0.22365 | C=COC(OCCO)OC1OCC(C2(C3CCCCO3)COCOC2)O1 |
| 0.22371 | C=COC(OCC1(C2CCCCO2)COCOC1)OC1CCCCO1 |
| 0.22384 | C=COC(OCC)OC1OCC(C2(C3CCCO3)COCOC2)O1 |
| 0.22418 | C=COC(OCCOC)OC1OCC(CC)O1 |
| 0.22423 | C=C(C)C(=O)OC1OCC(OC2CCCCO2)O1 |
| 0.22437 | C=COC(OCC)OC1(COC)COCOC1 |
| 0.22453 | C=C(C)C(=O)OCC1(C2CCCO2)COCOC1 |
| 0.22455 | C=CC(=O)OC1(C2OCC(CC)O2)COCOC1 |
| 0.22455 | C=CC(=O)OC1(C2COC(CC)O2)COCOC1 |
| 0.22508 | C=C(C)C(=O)OC1COC(OC2CCCCO2)O1 |
| 0.2251 | C=COC(OCC)OC1(C2COC(OC)O2)COCOC1 |
| 0.22516 | C=COC(OCCO)OCCOC1CCCCO1 |
| 0.22534 | C=COC(OCCO)OC1OCC(C2(OC)COCOC2)O1 |
| 0.22541 | C=COC(OCCO)OC1(C2COC(OC)O2)COCOC1 |
| 0.22562 | C=COC1OCC(C2(C3COC(CC)O3)COCOC2)O1 |
| 0.22566 | C=COC(OCCO)OCCCOC1CCCO1 |
| 0.2257 | C=COC(OCC)OC1(C2OCC(OC)O2)COCOC1 |
| 0.22581 | C=COC(OCCO)OC1COC(C2(CC)COCOC2)O1 |
| 0.22594 | C=CC(=O)OC1(C2OCC(OC)O2)COCOC1 |
| 0.22608 | C=COC1OCC(C2(OC)COCOC2)O1 |
| 0.22609 | C=COC(OCCO)OCCCOCC |
| 0.22628 | C=COC(OCCO)OC1COC(C2(OC)COCOC2)O1 |
| 0.22628 | C=COC(OCCO)OC1(C2OCC(OC)O2)COCOC1 |
| 0.22631 | C=COC(OCC1(OC)COCOC1)OC1CCCCO1 |
| 0.22631 | C=COC(OC1CCCCO1)OC1(COC)COCOC1 |

**[Table 2-4]**

| | |
|---|---|
| 0.22633 | C=COC(OCCCOC)OC1COC(CC)O1 |
| 0.22639 | C=COC(OCC)OC1OCC(C2(C3CCCCO3)COCOC2)O1 |
| 0.22656 | C=COC(OCCO)OCCCOC1CCCCO1 |
| 0.2267 | C=COC1(OC2(C3CCCCO3)COCOC2)COCOC1 |
| 0.2267 | C=COC1(OC2(C3CCCO3)COCOC2)COCOC1 |
| 0.2267 | C=COC(OCCOC)OC1(OC)COCOC1 |
| 0.2268 | C=COC(OCCO)OC1(OCCO)COCOC1 |
| 0.22689 | C=COC1OCC(OC2COC(OC)O2)O1 |
| 0.22699 | C=COC(OCCCOCCO)OC1CCCCO1 |
| 0.227 | C=C(C)C(=O)OC1(C2CCCO2)COCOC1 |
| 0.22701 | C=C(C)C(=O)OC1(OC2CCCO2)COCOC1 |
| 0.22708 | C=C(C)C(=O)OCC1 (C2CCCCO2)COCOC1 |
| 0.22716 | C=COC(OCCOCCO)OC1CCCCO1 |
| 0.22721 | C=CC(=O)OC1OCC(C2(CC)COCOC2)O1 |
| 0.22725 | C=COC(OCCO)OCCOCCO |
| 0.22742 | C=COC(OCC1CCCO1)OCC1CCCO1 |
| 0.22748 | C=CC(=O)OC1(OCCO)COCOC1 |
| 0.22754 | C=COC(OCCOC)OC1(CC)COCOC1 |
| 0.22762 | C=COC1OCC(C2(OCCO)COCOC2)O1 |
| 0.22762 | C=COC(OCC1CCCCO1)OCC1CCCCO1 |
| 0.22762 | C=COC(OCC1CCCCO1)OCC1CCCO1 |
| 0.22765 | C=COC(OC1CCCCO1)OC1(C2CCCCO2)COCOC1 |
| 0.2277 | C=COC(OCC)OC1COC(C2(OC)COCOC2)O1 |
| 0.22777 | C=COC1COC(C2(COC)COCOC2)O1 |
| 0.22785 | C=COC(OCCCOC1CCCCO1)OC1CCCCO1 |
| 0.2281 | C=COC(OCCOC)OCC1CCCCO1 |
| 0.22867 | C=COC(OCC)OCCCOCCO |
| 0.22884 | C=COC(OC1OCC(CC)O1)OC1OCC(CC)O1 |
| 0.22885 | C=COC(OCCOC)OC1(C2CCCCO2)COCOC1 |
| 0.22885 | C=COC(OCCOC)OC1(C2CCCO2)COCOC1 |
| 0.22886 | C=COC(OC1CCCO1)OC1OCC(C2(OC)COCOC2)O1 |
| 0.22914 | C=COC(OC1CCCCO1)OC1OCC(C2(OC)COCOC2)O1 |
| 0.22925 | C=CC(=O)OC1(C2COC(OC)O2)COCOC1 |
| 0.22934 | C=COCC1COC(C2(OC)COCOC2)O1 |
| 0.22936 | C=COC(OCCCOC)OC1(OC)COCOC1 |
| 0.22939 | C=COC1COC(C2(C3COC(CC)O3)COCOC2)O1 |
| 0.2294 | C=C(C)C(=O)OC1OCC(OC2CCCO2)O1 |
| 0.22941 | C=COC1COC(C2(C3COC(OC)O3)COCOC2)O1 |
| 0.22945 | C=COC(OCCCOC)OC1OCC(CC)O1 |

**[Table 2-5]**

| | |
|---|---|
| 0.22946 | C=CC(=O)OC1COC(C2(OC)COCOC2)O1 |
| 0.22951 | C=C(C)C(=O)OC1COC(OCCO)O1 |
| 0.22951 | C=C(C)C(=O)OC1OCC(OCCO)O1 |
| 0.22977 | C=COC(OCCO)OC1OCC(COC)O1 |
| 0.22993 | C=COC(OC1(OC)COCOC1)OC1(C2CCCCO2)COCOC1 |
| 0.22995 | C=COC(OC1(OC)COCOC1)OC1(C2CCCO2)COCOC1 |
| 0.23025 | C=C(C)C(=O)OC1COC(OC2CCCO2)O1 |
| 0.2303 | C=COC(OC1(CC)COCOC1)OC1(OC)COCOC1 |
| 0.23038 | C=COC(OCCCOC)OCC1CCCO1 |
| 0.23051 | C=COC1OCC(C2(COC)COCOC2)O1 |
| 0.23067 | C=CC(=O)OC1COC(C2(CC)COCOC2)O1 |
| 0.23079 | C=COC(OC1(CC)COCOC1 )OC1(CC)COCOC1 |
| 0.23104 | C=COC1(C2COC(COC)O2)COCOC1 |
| 0.23117 | C=COC1(C2OCC(C3(C4CCCCO4)COCOC3)O2)COCOC1 |
| 0.23117 | C=COC1(C2OCC(C3(C4CCCO4)COCOC3)O2)COCOC1 |
| 0.23117 | C=COC1(C2COC(C3(C4CCCCO4)COCOC3)O2)COCOC1 |
| 0.23117 | C=COC1(C2COC(C3(C4CCCO4)COCOC3)O2)COCOC1 |
| 0.23184 | C=CC(=O)OC1COC(C2(C3CCCO3)COCOC2)O1 |
| 0.23184 | C=COCC1OCC(C2(OC)COCOC2)O1 |
| 0.23194 | C=COC(OC1COC(OC)O1)OC1OCC(OC)O1 |
| 0.23214 | C=COC(OC1OCC(OC)O1)OC1OCC(OC)O1 |
| 0.23222 | C=C(C)C(=O)OC1(OC2CCCCO2)COCOC1 |
| 0.23234 | C=COC(OCC)OC1OCC(COC)O1 |
| 0.2326 | C=C(C)C(=O)OC1(C2CCCCO2)COCOC1 |
| 0.2326 | C=COC(OC1(C2CCCO2)COCOC1)OC1(C2CCCO2)COCO |
| 0.23274 | C=COC(OCCCOC)OC1(CC)COCOC1 |
| 0.2331 | C=COC(OC1(C2CCCCO2)COCOC1)OC1(C2CCCO2)COCO |
| 0.23373 | C=COC(OC1(CC)COCOC1)OC1(C2CCCCO2)COCOC1 |
| 0.23373 | C=COC(OC1(CC)COCOC1)OC1(C2CCCO2)COCOC1 |
| 0.23374 | C=COC(OCC)OCC1(OC)COCOC1 |
| 0.23384 | C=C(C)C(=O)OC1COC(C2(CC)COCOC2)O1 |
| 0.23404 | C=COC(OC1OCC(OC)O1)OC1(OC)COCOC1 |
| 0.23406 | C=COC(OC1COC(OC)O1)OC1(OC)COCOC1 |
| 0.23418 | C=COC1(C2OCC(COC)O2)COCOC1 |
| 0.23421 | C=COC(OC1COC(OC)O1)OC1COC(OC)O1 |
| 0.23438 | C=COC1COC(C2(C3OCC(OC)O3)COCOC2)O1 |
| 0.23477 | C=CC(=O)OC1OCC(C2(OC)COCOC2)O1 |
| 0.23481 | C=CC(=O)OC1COC(C2(C3CCCCO3)COCOC2)O1 |
| 0.23489 | C=C(C)C(=O)OC1(OCCO)COCOC1 |

**[Table 2-6]**

| | |
|---|---|
| 0.23567 | C=COC(OC1(C2CCCCO2)COCOC1)OC1(C2CCCCO2)COC |
| 0.23577 | C=COC(OC1CCCCO1)OC1OCC(C2(C3CCCCO3)COCOC2 |
| 0.23577 | C=COC(OC1CCCCO1)OC1COC(C2(C3CCCCO3)COCOC2 |
| 0.23583 | C=COC1OCC(C2(C3OCC(OC)O3)COCOC2)O1 |
| 0.236 | C=C(C)C(=O)OCC1(OC)COCOC1 |
| 0.23622 | C=COC(OC1(OC)COCOC1)OC1(OC)COCOC1 |
| 0.23642 | C=COC(OCC)OC1OCC(C2(OC)COCOC2)O1 |
| 0.23653 | C=COC(OCCCOC)OCCCOC |
| 0.23654 | C=COC(OCCCOC)OC1(C2CCCO2)COCOC1 |
| 0.23669 | C=C(C)C(=O)OC1(OCC)COCOC1 |
| 0.23687 | C=CC(=O)OC1OCC(C2(C3CCCO3)COCOC2)O1 |
| 0.23687 | C=COC(OCCCOC)OCC1CCCCO1 |
| 0.23708 | C=CC(=O)OC1OCC(C2(C3CCCCO3)COCOC2)O1 |
| 0.23834 | C=C(C)C(=O)OC1COC(C2(OC)COCOC2)O1 |
| 0.23834 | C=C(C)C(=O)OC1(C2OCC(OC)O2)COCOC1 |
| 0.23846 | C=COC(OCCO)OCCCOCCO |
| 0.23916 | C=C(C)C(=O)OC1(C2COC(CC)O2)COCOC1 |
| 0.23916 | C=C(C)C(=O)OC1(C2OCC(CC)O2)COCOC1 |
| 0.23919 | C=C(C)C(=O)OC1COC(C2(C3CCCCO3)COCOC2)O1 |
| 0.23975 | C=COC(OCCCOC)OC1(C2CCCCO2)COCOC1 |
| 0.24048 | C=C(C)C(=O)OC1(COC)COCOC1 |
| 0.24146 | C=C(C)C(=O)OC1OCC(C2(C3CCCCO3)COCOC2)O1 |
| 0.24146 | C=C(C)C(=O)OC1OCC(C2(C3CCCO3)COCOC2)O1 |
| 0.24252 | C=C(C)C(=O)OC1OCC(C2(OC)COCOC2)O1 |
| 0.24252 | C=C(C)C(=O)OC1(C2COC(OC)O2)COCOC1 |
| 0.24316 | C=COC1OCC(C2(C3COC(OC)O3)COCOC2)O1 |
| 0.24318 | C=COC(OCCCOC)OCCOC |
| 0.24798 | C=C(C)C(=O)OC1COC(C2(C3CCCO3)COCOC2)O1 |
| 0.24889 | C=COC1(C2OCC(C3(OC)COCOC3)O2)COCOC1 |
| 0.24889 | C=COC1(C2COC(C3(OC)COCOC3)O2)COCOC1 |

**[Table 2-7]**

| | |
|---|---|
| 0.2324 | C=C(C(=O)OCCC(OC)(OC)OC) |
| 0.2599 | C=C(C(=O)OCCOCC(OC)(OC)OC) |
| 0.242 | COCCCOC(=O)C=C(C(=O)OCCCOC) |
| 0.2309 | COCCOC(=O)C=C(C(=O)OCCOC) |
| 0.2458 | COCCOC(=O)C=C(C(=O)OCC1CCCO1) |

The machine learning method enabled generation of the regression formulas (regression models) to predict the physical property of the polymers.

Moreover, in the case where the interstitial water content was from 0.01 to 0.15 (Tables 1-1 to 1-3), the resulting polymers were found to adhere well to specific cells including cancer cells. Therefore, a polymer that has an interstitial water content of 0.01 to 0.15 as determined by the method for predicting a physical property of a polymer is excellent in selective adhesion to specific cells, particularly in selective adhesion to cancer cells. For example, a polymer produced by polymerizing a monomer expressed by any of the SMILES formats in Tables 1-1 to 1-3 had an interstitial water content of 0.01 to 0.15 and adhered well to cancer cells.

Further, in the case where the interstitial water content was from 0.22 to 0.5 (Tables 2-1 to 2-7), the resulting polymers were found to have good biocompatibility. Therefore, a polymer that has an interstitial water content of 0.22 to 0.5 as determined by the method for predicting a physical property of a polymer has excellent biocompatibility. For example, a polymer produced by polymerizing a monomer expressed by any of the SMILES formats in Tables 2-1 to 2-7 had good biocompatibility.

The above are the results in the case where a double bond was included and random forest regression calculation was used. In contrast, Table 3 shows predicted interstitial water contents obtained by a method including: constructing a prediction model using a monomer including no double bond with XGBoost regression calculation and similarly constructing a prediction model with CatBoost regression calculation (each hyperparameter is manually adjusted to reduce the root mean square error (RMSE)); constructing a prediction model using an Optuna framework with XGBoost regression calculation and adjusting the hyperparameters by Bayesian optimization; converting the monomers in the SMILES formats in Table 3 into monomers including no double bond (by cleaving a double bond and introducing a methyl group to each terminal); computing descriptors of the converted monomers; and applying the descriptors to the three prediction models to predict interstitial water contents. The predicted interstitial water contents were within a certain range and found to be somewhat plausible.

**[Table 3]**

| | Monomer including no double bond | | |
|---|---|---|---|
| | XGBoost | XGBoost | CatBoost |
| SMILES | Manual adjustment of hyperparameters | Bayesian optimization Adjustment of hyperparameters using Optuna framework | Manual adjustment of hyperparameters |
| C=C(C(=O)OCCCOCC) | 0.06302049 | 0.04934459 | 0.070548117 |
| C=C(C)(C(=O)OCC) | 0.10124662 | 0.07899422 | 0.089073982 |
| C=C(C)(C(=O)OCCO) | 0.08922913 | 0.08532548 | 0.112979359 |
| C=C(C(=O)OC1CCCO1) | 0.12442186 | 0.07899422 | 0.074042013 |
| C=C(OC1CCCCO1) | 0.07680637 | 0.083050214 | 0.086623772 |
| C=C(C(=O)OCC1CCCCO1) | 0.065671794 | 0.04934459 | 0.068652444 |
| C=C(OC(OCC)OCC) | 0.12361085 | 0.11269984 | 0.076005998 |
| C=C(C1OCCO1) | 0.079879634 | 0.07222404 | 0.092415444 |

Exemplary embodiments of the present invention include the following.

Embodiment 1. A method for predicting a physical property of a polymer, the method including:
Step 1 of obtaining a structure of a monomer from a structure of a polymer,
Step 2 of converting the obtained structure of the monomer or a converted structure of the monomer into a format recognizable by a computer,
Step 3 of computing a descriptor from the format recognizable by a computer,
Step 4 of obtaining a physical property of the polymer and performing a regression calculation using the physical property of the polymer as an objective variable and the descriptor as an explanatory variable to construct a regression model,
Step 5 of computing a format of a new monomer from the format recognizable by a computer,
Step 6 of computing a descriptor from the format of the new monomer or a converted format of the new monomer, and
Step 7 of applying the descriptor of the new monomer to the regression model to predict a physical property of a polymer produced by polymerizing the new monomer.

Embodiment 2. The method for predicting a physical property of a polymer according to Embodiment 1,
wherein the format recognizable by a computer is a SMILES format or a MOL file format.

Embodiment 3. The method for predicting a physical property of a polymer according to Embodiment 1 or 2,
wherein the regression calculation is at least one selected from the group consisting of multiple regression calculation, support vector regression calculation, decision tree calculation, random forest regression calculation, XGBoost regression calculation, LightGBM regression calculation, CatBoost regression calculation, Gaussian process regression calculation, neural networks, and deep learning.

Embodiment 4. The method for predicting a physical property of a polymer according to any combination with any one of Embodiments 1 to 3,
wherein the format of the new monomer is computed by at least one algorithm selected from the group consisting of BRICS, RECAP, and DOCS.

Embodiment 5. The method for predicting a physical property of a polymer according to any combination with any one of Embodiments 1 to 4,
wherein the physical property of a polymer is at least one selected from the group consisting of glass transition temperature, melting point, refractive index, contact angle, surface free energy, density, water absorption, interstitial water content, unfreezable water content, selective adhesion to specific cells, and biocompatibility.

Embodiment 6. The method for predicting a physical property of a polymer according to any combination with any one of Embodiments 1 to 5,
wherein the conversion of the structure of the monomer includes cleaving a carbon-carbon double bond in the structure of the monomer to form a multimer containing only carbon-carbon single bonds and introducing a methyl group to each terminal of the multimer to form a compound.

Embodiment 7. The method for predicting a physical property of a polymer according to any combination with any one of Embodiments 1 to 6,
wherein the Step 2 includes converting the structure of the monomer or a multimer converted from the structure of the monomer into a format recognizable by a computer.

Embodiment 8. A polymer having an interstitial water content of 0.01 to 0.15 as determined by the method for predicting a physical property of a polymer according to any combination with any one of Embodiments 1 to 7.

Embodiment 9. The polymer according to Embodiment 8, which is produced by polymerizing a monomer having a SMILES format in any of Tables 1-1 to 1-3.

Embodiment 10. The polymer according to Embodiment 8 or 9, which is capable of selectively adhering to specific cells.

Embodiment 11. The polymer according to Embodiment 10,
wherein the specific cells are cancer cells.

Embodiment 12. A polymer having an interstitial water content of 0.22 to 0.5 as determined by the method for predicting a physical property of a polymer according to any combination with any one of Embodiments 1 to 7.

Embodiment 13. The polymer according to Embodiment 12, which is produced by polymerizing a monomer having a SMILES format in any of Tables 2-1 to 2-7.

Embodiment 14. The polymer according to Embodiment 12 or 13, which has biocompatibility.

## Claims

1. A method for predicting a physical property of a polymer, the method comprising:
Step 1 of obtaining a structure of a monomer from a structure of a polymer,
Step 2 of converting the obtained structure of the monomer or a converted structure of the monomer into a format recognizable by a computer,
Step 3 of computing a descriptor from the format recognizable by a computer,
Step 4 of obtaining a physical property of the polymer and performing a regression calculation using the physical property of the polymer as an objective variable and the descriptor as an explanatory variable to construct a regression model,
Step 5 of computing a format of a new monomer from the format recognizable by a computer,
Step 6 of computing a descriptor from the format of the new monomer or a converted format of the new monomer, and
Step 7 of applying the descriptor of the new monomer to the regression model to predict a physical property of a polymer produced by polymerizing the new monomer.

2. The method for predicting a physical property of a polymer according to claim 1,
wherein the format recognizable by a computer is a SMILES format or a MOL file format.

3. The method for predicting a physical property of a polymer according to claim 1 or 2,
wherein the regression calculation is at least one selected from the group consisting of multiple regression calculation, support vector regression calculation, decision tree calculation, random forest regression calculation, XGBoost regression calculation, LightGBM regression calculation, CatBoost regression calculation, Gaussian process regression calculation, neural networks, and deep learning.

4. The method for predicting a physical property of a polymer according to any one of claims 1 to 3,
wherein the format of the new monomer is computed by at least one algorithm selected from the group consisting of BRICS, RECAP, and DOCS.

5. The method for predicting a physical property of a polymer according to any one of claims 1 to 4,
wherein the physical property of a polymer is at least one selected from the group consisting of glass transition temperature, melting point, refractive index, contact angle, surface free energy, density, water absorption, interstitial water content, unfreezable water content, selective adhesion to specific cells, and biocompatibility.

6. The method for predicting a physical property of a polymer according to any one of claims 1 to 5,
wherein the conversion of the structure of the monomer includes cleaving a carbon-carbon double bond in the structure of the monomer to form a multimer containing only carbon-carbon single bonds and introducing a methyl group to each terminal of the multimer to form a compound.

7. The method for predicting a physical property of a polymer according to any one of claims 1 to 6,
wherein the Step 2 includes converting the structure of the monomer or a multimer converted from the structure of the monomer into a format recognizable by a computer.

8. A polymer having an interstitial water content of 0.01 to 0.15 as determined by the method for predicting a physical property of a polymer according to any one of claims 1 to 7.

9. The polymer according to claim 8, which is produced by polymerizing a monomer having a SMILES format in any of Tables 1-1 to 1-3.

10. The polymer according to claim 8 or 9, which is capable of selectively adhering to specific cells.

11. The polymer according to claim 10,
wherein the specific cells are cancer cells.

12. A polymer having an interstitial water content of 0.22 to 0.5 as determined by the method for predicting a physical property of a polymer according to any one of claims 1 to 7.

13. The polymer according to claim 12, which is produced by polymerizing a monomer having a SMILES format in any of Tables 2-1 to 2-7.

14. The polymer according to claim 12 or 13, which has biocompatibility.
